# EUROPEAN PATENT APPLICATION

(11) **EP 3 323 880 A1**
(43) Date of publication of application: **23.05.2018**
(21) Application number: 16836577.3
(22) Date of filing: 09.08.2016
(51) Int. Cl.: C12M 3/02

(54) **CULTURE DEVICE FOR TISSUE CELL SUSPENSION**

(30) Priority: 18.08.2015 CN 201510508053
(71) Applicant: Chongqing Runze Pharmaceutical Co. Ltd., Chongqing 400042 (CN)
(72) Inventor: YE, Lei, Chongqing 400042 (CN)
(74) Representative: Inal, Aysegul Seda
(86) International application number: PCT/CN2016/094169
(87) International publication number: WO 2017/028713

(57) **Abstract**

The present invention relates to a culture device for tissue cell suspension. The culture device includes a tissue cell culture body. The tissue cell culture body is a porous material. The porous material is formed by cavities classified into different levels according to the pore size of material and cavity walls surrounding to form the cavities. A lower level of small cavities is provided to surround the cavity wall which forms the upper level of large cavity. Cavities of each level are in communication with each other, and cavities between respective levels are also in communication with each other. The culture device further includes a swirler device. Such culture device can particularly facilitate normal and unrestricted growth of the suspension of cells in three-dimensional space, obstructing the formation of over-dense cell region or nutrient-rich region during the cell culturing, enabling a uniform cell distribution, and facilitating normal growth of cells.

## Description

### TECHNICAL FIELD

The present invention relates to a tissue cell culture device, particularly to a tissue cell culture device which facilitates suspension of cells to grow normally and unrestrictedly in the culture solution in the culture environment.

### BACKGROUND

The term "cell culture" refers to a culture technology as follows: cells are taken from the body tissue; an internal environment of the body is simulated; under a sterilized condition at a suitable temperature, a suitable pH value, and certain nutrition, cells can grow and proliferate in vitro, maintaining their structures, functions, and physiological properties. Cell culture can be classified into two general categories, i.e., adherent growth and suspension growth, based on whether the cells can grow adherently along a substrata in the culture utensil. During the culture, adherent cells can grow adherently along the surface of the substrata. Usually, fibers, microcarriers, porous material, etc. can be used as the culture body. Cells are adherently cultured on the culture body. On the other hand, regarding the suspension tissue cells, since they do not grow adherently along the substrata, usually, the culture body is not required during the culture. Rather, the suspension tissue cells are cultured directly in the culture solution. The culture device for such kind of cells mainly includes culture dish (flask) and stirring device. For example, patent CN201224734Y discloses a "cell suspension culture flask with two stirring rods", wherein the cell suspension culture flask with two stirring rods includes a bottle body, a bottle cap , and a stirring device. One end of the stirring device is connected to an inner side of the bottle cap. The other end of the stirring device extends into the bottle body to approach the bottom end of the bottle body. The stirring device is composed of a fixing rod, an upper connection flexible tube, a shaft rod, a fork-shaped rotor part, a lower connection flexible tube, and a magnetic rod. The upper end of the fixing rod is fixedly connected to the center of the inner side of the bottle cap. The lower end of fixing rod is connected to the upper end of the shaft rod through the upper connection flexible tube. At the lower end of each fork of the fork-shaped rotor part, the fork-shaped rotor part is connected to the magnetic rod through the flexible tube. The culture flask can be used to culture cells of bacteria, fungus, plants, etc. in suspension, and is particularly suitable for culturing mammal cells and insect cells in suspension. CN 201762335U discloses a "high-density cell suspension culture device", which includes a culture flask, a magnetic stirring part disposed at the bottom of the flask, and a low-speed regulated magnetic stirrer. The top end of the culture flask and two sides of the upper end of the culture flask are provided with openings. The culture flask is disposed above the low-speed regulated magnetic stirrer. The magnetic stirring part disposed at the bottom of the culture flask is controlled to rotate by the magnetic stirrer, so as to culture the suspension of cells. However, when cells are cultured with the above prior art, as the density of cells increases, uniform distribution of cells cannot be achieved, resulting in local over-dense cell regions, affecting the transmission of the culture solution. Since the concentration of culture solution is uneven, it obstructs the normal growth of cells. Moreover, the high density of cells will slow down the growth rate of cells. The stirring method of the above prior art has a large sheer force, which may cause damage to cells, obstructing the culture of cells.

### SUMMARY OF THE INVENTION

The purpose of the present invention is to overcome the defects of the prior art, and to provide a tissue cell culture device which facilitates cells to grow normally and unrestrictedly with a uniform distribution in the culture solution, making cells grow normally.

The defects of the prior art can be overcome if a cell culture device as follows is provided. The device makes cells distribute uniformly through a series of uniform mesh space. Meanwhile, during the growth and proliferation of cells, as the number of cells increases gradually such that cells are getting denser, there is a power source facilitating the free flow of nutrient solution or metabolite, to ensure normal growth of cells. The sheer force posed on cells is reduced by the flow of solution and mesh protection.

The purpose of the present invention can be achieved by the following technical solutions:
A culture device for tissue cell suspension includes a tissue cell culture body, and the tissue cell culture body is a porous material. The porous material is formed by cavities classified into different levels according to the pore size of material and cavity walls surrounding to form the cavities. A lower level of small cavities are provided on the cavity wall which arounded forms an upper level of large cavities. Cavities of each level are in communication with each other, and cavities within respective levels are also in communication with each other. The culture device further includes a swirler device, which can make the culture solution in the culture device generate a swirling vortex.

In the above tissue cell culture device, cavities of the porous material used as the tissue cell culture body, that are classified based on material pore size, are classified into at least more than two levels. The next level of small cavities is provided on the cavity wall which enclosed forming the upper level of large the cavities; or a lower level of respective level of small cavities are provided on the cavity wall which encloses, forming the upper level of large cavities; or any combination of lower level of respective level of small cavities is provided on the cavity wall which encloses, forming the upper level of large cavities.

When the cavities of the porous material are classified into three levels, the next level of small cavities are provided on the cavity wall which encloses, forming an upper level of large cavities; or two lower levels of small cavities are provided on the cavity wall which encloses forming the upper level of large cavities. Certainly, cavity walls of the lower level small cavities are all formed by enclosing the next lower level of small cavities. Similarly, regarding the cavities of porous material that are classified into more levels, the cavity wall of each level of large cavities can be provided with only one next level of small cavities. Also, a plurality of lower levels of small cavities can be provided on the cavity wall which encloses forming the upper level of large cavities. Otherwise, any combination of a plurality of lower levels of small cavities are provided on the cavity wall which encloses forming the upper level of large the cavities. The technical solution is optimal when the cavities of the porous material are classified into three levels. Also when the cavities of the porous material are classified into three levels, the structure in which the next level of small cavities are provided on the cavity wall surrounding to form the upper level of large cavities is optimal.

Furthermore, the size of the lower level of cavities of the porous material is nanoscale, while the sizes of other levels of cavities are at least more than an average diameter of the culture cells.

Furthermore, when cavities of the porous material are classified into three levels, the next level of small cavities are provided on the cavity wall which encloses forming an upper level of large cavities. The pore size of the lowest level of cavities is nanoscale. The pore size of the cavity which is one level upper than the pore size of the lowest level of cavities is at least more than twice of an average diameter of the culture cells. It is optimal that the pore size of the cavity which is two levels upper than the pore size of the lowest level of small cavities is at least more than four times of an average diameter of the culture cells.

The porous material as the tissue cell culture body of the present invention can be prepared by the following preparation method:

### (1) Material preparation

The raw material powder is mixed with the pore-forming agent which is used to prepare the lowest level of pores, and a slurry is prepared by the mixed raw material powder and the pore-forming agent.

The slurry is filled uniformly into a high polymer material substrata to form a green body. The green body is dried and crushed, so as to obtain mixed grains including the raw material, the pore-forming agent, and the high polymer material substrata material.
(2) The above obtained mixed grains are mixed uniformly with the pore-forming agent which is used to prepare an upper level of pores which are larger than the lowest level of pores, so as to prepare a compact green body.
(3) The compact green body is sintered in vacuum. The sintered green body is subjected to a regular follow-up treatment based on raw material process, so as to obtain the porous material.

More specifically, in the preparation method of the porous material, before preparing the compact green body, the mixed grains are mixed uniformly with the pore-forming agent which is used to prepare the pores that are one level upper than the lowest level of pores. The mixture of the mixed grains and the pore-forming agent is poured uniformly into the high polymer material substrata. The pore size of the high polymer material substrata is larger than the grain size of mixed grains and the grain size of the pore-forming agent. The strut is used as the pore-forming agent for pores that are two levels upper than the lowest level of pores. As such, after vacuum sintering, the multilevel pore material including three levels of pores can be prepared. Similarly, porous material with more levels of pores can be prepared.

More specifically, in the preparation method of the porous material, pores of the high polymer material substrata whose pore size is larger than the grain size of mixed grains and the pore size of the pore-forming agent are three-dimensional transfixion. Thus, the prepared pores are also three-dimensional transfixion.

Further, the above culture device for tissue cell suspension further includes culture dish accommodating the tissue cell culture body. The tissue cell culture body is fixed in the culture dish. The swirling device is provided below the tissue cell culture body.

The above swirler device includes multiple groups of air transmission pipes supported by the holder. Each of the multiple groups of air transmission pipes is provided with an outlet pipe and an intake pipe. The intake pipe is connected to the control valve outside the culture dish. The control valve is also connected to a main intake pipe.

The above air transmission pipe is annular. The annular air transmission pipe is provided with the outlet pipe. The projection of an axial line of the outlet pipe in the horizontal plane is tangential with respect to the projection of an axial line annular of the air transmission pipe in the horizontal plane.

It is optimal that the above multiple groups of air transmission pipes have even groups, such as two groups, four groups, six groups or more. Moreover, on the projection in the horizontal plane, if the direction of one group of outlet pipes is the same as the clockwise tangential direction of the axial line of the air transmission pipe, the direction of the other group of outlet pipes is the same as the anti-clockwise tangential direction of the axial line of the air transmission pipe, and vice versa.

Further, the angle of each group of outlet pipes with respect to the horizontal plane is less than or equal to 45°.

Further, the exit of the above outlet pipe is provided with a screen mesh or a membrane with pores. Advantages of the present invention are as below:
1. The present invention provides a tissue cell culture device which facilitates normal and unrestricted growth of the tissue cell suspension in three-dimensional space. The porous material is used as the cell culture body. Particularly, in the porous material, cavities that are classified according to the pore size of the material and the cavity wall surrounding to form the cavity are reasonably designed, such that the culture space is divided into a series of three-dimensional uniformly-distributed grid space. The smallest pores are used to transfer nutrient solution and metabolite. Large pores are used as the growth space for the cells. For any cavity, due to the restriction of its size, the over-density of cells can be avoided. When a certain intensity is reached, cells will move to other cavities. Since cavities of each level are connected with each other and cavities between respective levels are also connected with each other, cells can move freely between large pores, providing conditions for the cells to go through therein freely. During the cell culturing, the cell culture body with such structure obstructs forming of over-dense cell regions or nutrient-rich region, enabling a uniform cell distribution, and facilitating normal growth of cells.
2. In the tissue cell culture device of the present invention, since cavities of each level are connected to each other and cavities between respective levels are also connected to each other, and due to the existence of pores of nanoscale, nutrient solution or metabolite can go through freely. Even if the number of cells increases, cavities are getting smaller or blocked, nutrient solution or metabolite will not be obstructed from freely infiltrating (or referred as permeating) in the cell culture body, such that the smooth and normal growth of the cells can be ensured.
3. In the tissue cell culture device of the present invention, cavities of the porous material are configured into different levels. Moreover, the number of levels of respective level of cavities and the configuration on the cavity wall of respective level of cavities are designed reasonably, so as to meet various growth conditions of different types of tissue cells. Particularly, when the pores are divided into three levels, the pore size of the third level of small cavities is of nanoscale pores. The pore size of the second level of cavities is at least more than twice of an average diameter of the culture cells. The pore size of the first level of cavities is at least more than four times of an average diameter of the culture cells. Such design is particularly beneficial for the growth of cells, ensuring that the cells can grow in the second level of cavities and move freely in the first level of cavities. The pore size of the lowest level of cavities is nanoscale pore. Small pores will generate capillary force, which may accelerate the flow of culture solution and metabolite. Cells can be sucked into the cell culture body, such that the cells are facilitated to move within the tissue cell culture body. Moreover, the capillary force also acts on the cells through the culture solution, so as to promote the growth of the cells. Efficient and massive culture of cells can be achieved. Thus, the present invention has significant advantages compared to the existing cell suspension culture technology.
4. The preparation method of the porous material as tissue cell culture body provided by the present invention can effectively control the pore size, the configuration of pores, the connectivity, and the uniformity of the distribution of pores. The method is simple, convenient, and easy to achieve. Parameters are easy to adjust and control.
5. With multiple groups of intake pipes, air transmission pipes, and outlet pipes, in combination with the valve body controlling, the intake air can form a swirling flow in the culture solution in a clockwise or an anti-clockwise rotating direction. Thus, the efficient stirring of the culture solution can be achieved. The unevenness of the culture solution can be avoided. With the flow of the fluid and the protection of the cell culture body, the shear force impacted on the cells is reduced. Since, the outlet pipe is provided at an angle with respect to the horizontal plane, the effect of the culture solution on the above cell culture body is more significant.
6. The exit of the outlet pipe is provided with a screen mesh or a membrane with pores, which reduces the bubbles, so as to prevent large bubbles from cracking or damaging the cells.

### BRIEF DESCRIPTION OF THE DRAWINGS

Hereinafter, with reference to drawings and embodiments, the present invention is further illustrated.

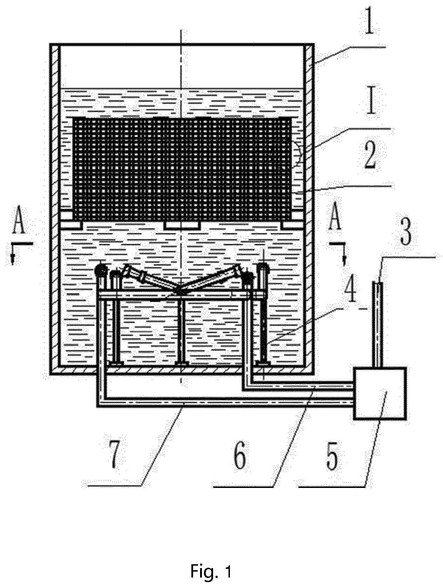

Figure 1 is a schematic diagram of the tissue cell culture device of the present invention.

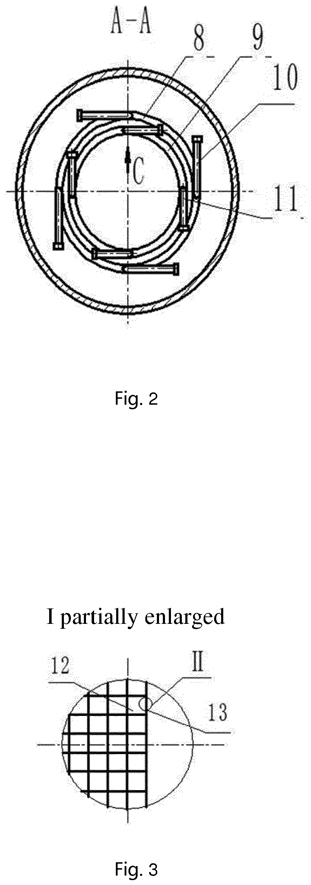

Figure 2 is a sectional diagram taken along A-A in Figure 1.
Figure 3 is a partially enlarged view of the structure of the tissue cell culture body in Figure 1.

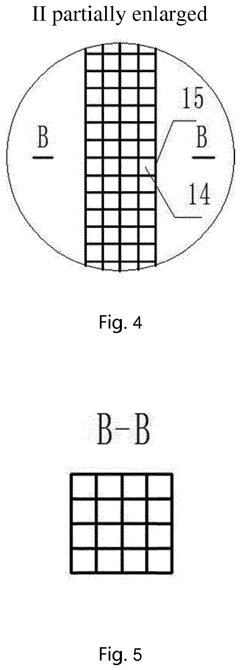

Figure 4 is a partially enlarged diagram of the cavity wall of the tissue cell culture body in Figure 3.
Figure 5 is a sectional diagram taken along B-B in Figure 4.

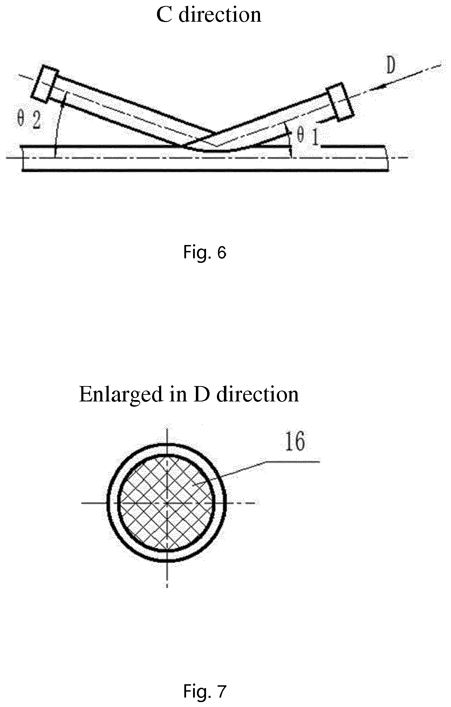

Figure 6 is a partial view taken in the direction of C in Figure 2.
Figure 7 is a partially enlarged view taken in the direction of D in Figure 6.

### DETAILED DESCRIPTION OF THE INVENTION

Hereinafter, with reference to drawings, embodiments of the present invention are described. With the technical solution of the present invention as premises, embodiments provide detailed embodiments and specific operation procedure. However, the protective scope of the present invention is not limited to the following embodiments.

In the drawings; 1 is the culture dish; 2 is the cell culture body; 3 is the main intake pipe; 4 is the holder supporting the air transmission; 5 is the control valve; 6 is the first intake pipe; 7 is the second intake pipe; 8 is the second air transmission pipe; 9 is the first air transmission pipe; 10 is the second outlet pipe; 11 is the first outlet pipe; 12 is the cavity of the tissue cell culture body; 13 is the cavity wall of the cavity; 14 is the cavity on cavity wall 13; 15 is the cavity wall of cavity 14; 16 is a screen mesh or a membrane with pores installed on an exit of the first outlet pipe 11 or the second outlet pipe 10.

Embodiments of the present invention are provided in detail as below:

### Embodiment 1:

Referring to the drawings, a culture device for tissue cell suspension is provided. The culture device includes a tissue cell culture body 2. The tissue cell culture body 2 is a porous tantalum material. The porous tantalum material is formed by cavities classified into different levels according to the pore size of the material and cavity walls surrounding to form the cavities. A lower level of small cavity is provided on the cavity wall which are enclosed to form the upper level of large cavity. Cavities of each level are connected with each other, and cavities between respective levels are also connected with each other. The culture device further includes a swirler device provided therein.

Particularly in the embodiment, the porous tantalum material includes three levels of cavity, wherein cavity wall 13 of a second upper level of large cavities 12 (hereinafter, referred as the first level of cavities), that are uniformly distributed and are connected with each other, is provided with a first upper level of large cavities 14 (hereinafter, referred as the second level of cavities) that are uniformly distributed and are connected with each other. Cavity wall 15 of the second level of cavities 14 is provided with a lower level of small cavities (hereinafter, referred as the third level of cavities) that are uniformly distributed and are connected with each other. Cavities between respective levels are also connected with each other. The pore size of the first level of cavities 12 is 160µm-650µm. The pore size of the second level of cavities 14 is 60µm-90µm. The pore size of the third level of cavities is 400nm-700nm.

The preparation method of the porous tantalum material is as below:

### (1) Material preparation

Tantalum powder with a grain size of 1µm-10µm is used as the raw material. Urea with a grain size of 450nm-780nm is used as the pore-forming agent of the lowest level of pores. Polystyrene with a grain size of 450nm-780nm is used as the adhesive. The slurry is prepared based on a volume ratio of 1: 2: 1:7.5 based on tantalum powder: urea: polystyrene: distilled water.

Polyester foam with a size of 100µm-200µm is used. The slurry is filled therein uniformly with the foam impregnation method to form a green body. The green body is dried. Next, the green body is crushed, so as to obtain mixed grains with a grain size of 65µm-100µm, including the raw material, the pore-forming agent, and the polyester foam.
(2) The mixed grains and ethyl cellulose with a grain size of 65µm-100µm are mixed uniformly based on a volume ratio of 2: 1. The mixture is poured uniformly into the polyester foam with a strut diameter of 200µm-720µm and a size of 400µm-600µm, which is three-dimensional transfixion. Next, the polyester foam is disposed into a closed mold to be pressed into a compact green body.
(3) The compact green body is sintered in vacuum. The sintered green body is subjected to a regular follow-up heat treatment based on tantalum material process, so as to obtain the porous tantalum with three levels of pores.

Further referring to the drawings, it can be seen that the tissue cell culture device of the present embodiment further includes culture dish 1 accommodating the tissue cell culture body. The tissue cell culture body 2 is fixed in the culture dish 1. The swirler device is provided below the tissue cell culture body.

The swirler device includes two groups of air transmission pipes supported by holder 4. The air transmission pipes are annular. That is, in the Figure, the first air transmission pipe 9 and the second air transmission pipe 8 both are supported by holder 4. The first air transmission pipe 9 and the second air transmission pipe 8 are evenly provided with the first outlet pipe 11 and the second outlet pipe 12, that are connected with the first air transmission pipe 9 and the second air transmission pipe 8 respectively. The projection of the axial line of the first outlet pipe 11 and that of the second outlet pipe 12 in the horizontal plane are respectively tangential with respect to the projection of the axial line of the first air transmission pipe 9 and that of the second air transmission pipe 8 in the horizontal plane. The first intake pipe 6 and the second intake pipe 7 respectively are connected with the first air transmission pipe 9 and the second air transmission pipe 8 go through the culture dish 1 to be connected to control valve 5. Main intake pipe 3 is connected to control valve 5. Control valve 5 can control the air to go through either the first intake pipe 6 or the second intake pipe 7 at the same time. In the projection of the horizontal plane, the direction of the first outlet pipe 11 is the same as the clockwise tangential direction of the axial line of the first air transmission pipe ring 9, while the direction of the second outlet pipe 12 is the same as the anti-clockwise tangential direction of the axial line of the second air transmission pipe ring 8.

θ1 and θ2 that are angles of the first outlet pipe 11 and the second outlet pipe 12 with respect to the horizontal plane are 30°.

Exits of the first outlet pipe 11 and the second outlet pipe 12 are provided with screen mesh 16. The average size of screen mesh 16 is 0.5mm.

During the cell culturing, DMEM (Dulbecco's Modified Eagle Medium) culture solution is used. S180 sarcoma cells as the culture cells are disposed into tissue cell culture body 2. Next, tissue cell culture body 2 is disposed into culture dish 1. The first intake pipe 6 and the second intake pipe 7 alternately take in air respectively, 3 minutes each time.

The culture results show that the body of the cultured cell is full, and the growth state is good.

### Embodiment 2:

Cell culture body 2 of the tissue cell culture device of the present embodiment uses porous silicon dioxide material, which has two levels of cavities. Cavity wall 13 of an upper level of large cavities 12, that are uniformly distributed and are connected with each other, is provided with a lower level of small cavities 14 that are uniformly distributed and are connected with each. Two levels of cavities are also connected with each other. The pore size of the upper level of large cavities 12 is 20µm-80µm. The pore size of the lower level of small cavity 14 is 100nm-300nm. θ1 and θ2 that are angles of the first outlet pipe 11 and the second outlet pipe 12 with respect to the horizontal plane are 15°. Exits of the first outlet pipe 11 and the second outlet pipe 12 are provided with a membrane with pores 16, the average size of which is 0.3mm.

The preparation method of such kind of porous silicon dioxide material is as below:

### (1) Material preparation

Silicon dioxide powder with a size of 1µm-10µm is used. Urea with the grain size of 130nm-350nm is used as the pore-forming agent of the lowest level of pores. The silicon dioxide powder and the urea are mixed uniformly. Starch with a size of 130nm-350nm is used as the adhesive. The slurry is prepared based on a volume ratio of 1: 1. 5: 1: 7 based on silicon dioxide powder: urea: starch: distilled water.

The slurry is filled uniformly into polyester foam with a strut diameter of 25µm-90µm with the foam impregnation method, so as to form a green body. The green body is dried. Next, the green body is crushed, so as to obtain mixed grains with a grain size of 25µm-90µm, including the silicon dioxide powder, the pore-forming agent, and polyester foam.
(2) The mixed grains and methyl cellulose with a grain size of 25µm-90µm are mixed uniformly based on a volume ratio of 2: 1. Next, the mixture is disposed into a closed mold to be pressed into the compact green body.
(3) The compact green body is sintered in vacuum. The sintered green body is subjected to regular follow-up heat treatment based on silicon dioxide process, so as to obtain the porous silicon dioxide with two levels of pores.

Remaining steps are the same as those in Embodiment 1.

During the cell culturing, BME (Basal Medium Eagle) culture solution is used. Lymph cells as the culture cells are disposed into tissue cell culture body 2. Next, tissue cell culture body 2 is disposed into culture dish 1. The first intake pipe 6 and the second intake pipe 7 alternately take in air respectively, each time 5 minutes.

The culture results show that the body of the cultured cell is full, and the growth state is good.

### Embodiment 3:

Cell culture body 2 of the tissue cell culture device of the present embodiment uses porous titanium material, which includes three levels of cavities. Cavity wall 13 of a second upper level of large cavities 12, (hereinafter, referred as the first level of cavity) that are uniformly distributed and are connected with each other, is provided with a first upper level of large cavities 14 (hereinafter, referred as the second level of cavity) that are uniformly distributed and are connected with each other. Cavity wall 15 of the upper level of large cavities is provided with a lower level of small cavities (hereinafter, referred as the third level of cavities), that are uniformly distributed and are connected with each other. Cavities between respective levels are also connected with each other. The pore size of the first level of cavities 12 is 300µm-700µm. The pore size of the second level of cavities 14 is 60µm-95µm. The pore size of the third level of cavities is 400nm-700nm. θ1 and θ2 that are angles of the first outlet pipe 11 and the second outlet pipe 12 with respect to the horizontal plane are 45°. Exits of the first outlet pipe 11 and the second outlet pipe 12 are provided with a membrane with pores 16, the average size of which is 0.7mm.

The preparation method of the porous titanium material is as below:

### (1) Material preparation

Titanium powder grain with a size of 1µm-10µm is used as the raw material. The urea with a grain size of 430nm-750nm is used as the pore-forming agent of the lowest level of pores. Polystyrene with a grain size of 430nm-750nm is used as the adhesive. The slurry is prepared based on a volume ratio of 1: 2: 1: 7. 5 based on titanium powder: urea: polystyrene: distilled water.

Polyester foam with a size of 100µm-200µm is used, and the slurry is filled therein uniformly with the foam impregnation method to form a green body. The green body is dried, Next, the green body is crushed, so as to obtain mixed grains with a grain size of 65µm-105µm, including the raw material, the pore-forming agent, and the polyester foam.
(2) The mixed grains and ethyl cellulose with a grain size of 65µm-10µm are mixed uniformly based on a volume ratio of 2: 1. The mixture is uniformly into the polyester foam strut diameter of 350µm-780µm and a size of 400µm-600µm, which is three-dimensional communicating. Next, the polyester foam is disposed into a closed mold to be pressed into a compact green body.
(3) The compact green body is sintered in vacuum. The sintered green body is subjected to regular follow-up heat treatment based on titanium material process, so as to obtain porous titanium with three levels of pores.

Remaining steps are the same as those in Embodiment 1.

During the cell culturing, MEM (Minimum Essential Medium) culture solution is used. Culture cells K562 are disposed into tissue cell culture body 2. Next, tissue cell culture body 2 is disposed into culture dish 1. The first intake pipe 6 and the second intake pipe 7 alternately take in air respectively, 4 minutes each time.

The culture results show that the body of the cultured cell is full, and the growth state is good.

## Claims

1. A culture device for tissue cell suspension, comprising:
a tissue cell culture body;
wherein
the tissue cell culture body is a porous material;
the porous material is formed by cavities classified into different levels according to a pore size of material and cavity walls surrounding to form the cavities;
a lower level of small cavities is provided to surround the cavity walls which forms an upper level of large cavities;
cavities of each level are in communication with each other;
cavities among different levels are also in communication with each other;
the culture device further includes a swirler device disposed therein.

2. The culture device for tissue cell suspension of claim 1, wherein
the plurality of levels comprise at least two levels;
a next level of the plurality of the small cavities are provided on the plurality of cavity walls forming the upper level of large the cavities;
or
the lower level comprises a plurality of subordinate levels,
the subordinate levels of the plurality of small cavities is provided on the plurality of cavity walls forming the upper level of large cavities;
or
any combination of the subordinate level of the plurality of small cavities are provided on the cavity wall forming the upper level of large cavities.

3. The culture device for tissue cell suspension of claim 1, **characterized in that**
cavities of the porous material as the tissue cell culture body that are classified based on material pore size are classified into three levels;
wherein a next level of small cavities is provided to surround the cavity wall forming the upper level of large the cavities;
or
two lower levels of small cavities are provided to surround the cavity wall forming the upper level of large cavities.

4. The culture device for tissue cell suspension of any one of claims 1-3, **characterized in that**
regarding the porous material as the tissue cell culture body, a pore size of the lower level of cavities is nanoscale, and a pore size of other levels of cavities is at least more than an average diameter of the culture cells.

5. The culture device for tissue cell suspension of any one of claims 1-3, **characterized in that**
regarding the porous material as the tissue cell culture body, a pore size of the lower level of cavities is nanoscale, and a pore size of other levels of cavities is at least more than twice of an average diameter of the culture cells.

6. The culture device for tissue cell suspension of claim 3, **characterized in that**
regarding the porous material as the tissue cell culture body, a next level of small cavities are provided to surround the cavity wall forming an upper level of large cavities;
the pore size of the lowest level of cavities is nanoscale;
a pore size of the cavity which is one level upper than the pore size of the lowest level of cavities is at least more than twice of an average diameter of the culture cells;
a pore size of the cavity which is two levels upper than the pore size of the lowest level of small cavities is at least more than four times of an average diameter of the culture cells.

7. The culture device for tissue cell suspension of any one of claims 1-6, **characterized in that**
the culture device further includes a culture dish accommodating the tissue cell culture body;
the tissue cell culture body is fixed in the culture dish;
the swirler device is provided below the tissue cell culture body.

8. The culture device for tissue cell suspension of claim 7, **characterized in that**
the swirler device includes multiple groups of air transmission pipes supported by the holder;
each of the multiple groups of air transmission pipes is provided with an outlet pipe and an intake pipe;
the intake pipe is connected to the control valve outside the culture dish;
the control valve is also connected to a main intake pipe.

9. The culture device for tissue cell suspension of claim 8, **characterized in that**
the air transmission pipe is annular;
the annular air transmission pipe is provided with an outlet pipe;
a projection of an axial line of the outlet pipe in the horizontal plane is tangential with respect to a projection of an axial line of the annular air transmission pipe in the horizontal plane.

10. The culture device for tissue cell suspension of claim 8 or 9, **characterized in that**
the multiple groups of air transmission pipes are even groups of air transmission pipes;
on the projection of the horizontal plane, a direction of one group of outlet pipes is the same as a clockwise tangential direction of the axial line of the air transmission pipe, while a direction of the other group of outlet pipes is the same as an anti-clockwise tangential direction of the axial line of the air transmission pipe.

11. The culture device for tissue cell suspension of any one of claims 8-10, **characterized in that** an angle of the outlet pipe with respect to the horizontal plane is less than or equal to 45°.

12. The culture device for tissue cell suspension of any one of claims 8-11, **characterized in that** an exit of the outlet pipe is provided with a screen mesh or a membrane with pores.
